# EUROPEAN PATENT APPLICATION

(11) **EP 4 470 605 A1**
(43) Date of publication of application: **04.12.2024**
(21) Application number: 22924261.5
(22) Date of filing: 28.01.2022
(51) Int. Cl.: A61N 2/00, A61N 2/02, A61N 1/36, A61F 7/00

(54) **ARTHRITIS PAIN TREATMENT DEVICE AND DRIVING METHOD THEREFOR**

(30) Priority: 27.01.2022 KR 20220012381
(71) Applicant: Entwick Inc., Yongin-si, Gyeonggi-do 16914 (KR)
(72) Inventor: JEONG, Jae Joon, Seoul 03323 (KR)
(74) Representative: Cabinet Chaillot
(86) International application number: PCT/KR2022/001554
(87) International publication number: WO 2023/146003

(57) **Abstract**

The present invention relates to an arthritis pain treatment device and a method of driving the treatment device. An arthritis pain treatment device according to an embodiment of the present invention is an arthritis pain treatment device that is worn on a body joint of a user, and may include: a pulsed electromagnetic generator that operates to activate cells of a body joint, generates a magnetic field to the body joint, and changes polarity of the magnetic field; a low-level laser generator that operates to reduce inflammation at the body joint, generates light of a predetermined wavelength to the body joint, and changes brightness of the light; an electric muscle stimulation generator that operates to induce contraction and relaxation of muscles of the body joint and applies current to the muscles of the body joint; a heat generator that generates heat to the muscles around the body joint in order to generate flow of blood around the body joint; and a controller that controls the pulsed electromagnetic generator, the low-level laser generator, the electric muscle stimulation generator, and the heat generator in accordance with instructions from a user.

## Description

### [Technical Field]

The present invention relates to an arthritis pain treatment device and a method of driving the treatment device, and more particularly, to an arthritis pain treatment device that transmits a magnetic force to a deep joint inflamed portion in a bone in a small magnitude by applying a coil-type pulsed electromagnetic generator, enhances the depth of penetration by controlling a low-level laser in a pulse control type, and improves muscle fatigue around a joint and increases muscle strength around a joint by applying an electric muscle stimulation generator and a heat generator, thereby providing a complex function for treating an arthritis pain; and a method of driving the treatment device.

### [Background Art]

A joint is composed of a cartilage, a joint capsule, a synovial membrane, ligaments, tendons, muscles, etc., to allow smooth movement between bones, and serves to absorb shocks that are generated by movement. In particular, when the cartilage at the point where bones meet become thin due to contact, the friction of the bones increases, and inflammation and pain are generated, which is called arthritis. Various devices are being introduced for such treatment of arthritis.

In the related art, there have been disclosed methods and devices for treating arthritis using ultrasonic waves. There is an ultrasonic device designed to generate ultrasonic waves of 200~800mW/cm² to apply low-intensity ultrasonic energy of 100~400mW/cm² to cartilage cells deep in the skin for the treatment or prevention of degenerative arthritis. However, when ultrasonic vibration collides with rigid materials such as bones, the intensity decreases, whereby there is a problem that the stimulation may not be transmitted to the cartilage cells between bones.

Further, a method of treating arthritis and a complex electromagnetic treatment device that use a pulsed electro-magnetic field have been disclosed in the related art. A Pulsed Electro-Magnetic Field (PEMF) therapy, which helps regeneration of cells by applying a pulsed electromagnetic field to an affected area, has been widely used since its approval by the U.S. Food and Drug Administration in 1979 for treatment of fractures relating to malunion. However, because it forms a loop wire-type wire and applies current in the form of frequency, in order to generate a sufficient magnetic force, a relatively large loop wire, high voltage, and high current are required, which lead to the problem of increasing the size of treatment devices.

Furthermore, in the related art, treatment methods for arthritis and low-power laser joint treatment devices using a low-level laser have also been introduced. A low-level laser (LLL) therapy irradiates an affected area with infrared light of 600 ~ 950nm to promote cellular metabolism and reduce inflammation, but reflection and dispersion are generated and penetration depth is low due to the characteristic of light. There is a problem in particular that it is impossible to penetrate opaque materials such as bones.

Lastly, therapies using electrical muscle stimulation and low-frequency joint treatment devices have been introduced in the related art. Electrical muscle stimulation (EMS) is widely used for muscle strengthening and massage functions through muscle contraction and relaxation by transmitting a low-frequency current directly to muscles, rather than being a therapy for arthritis. However, low-frequency EMS treatment devices primarily stimulate and massage muscles, and have a problem that they cannot relieve joint inflammation.

Summarizing the technologies of the related art again, existing arthritis treatment devices have limitations in treating inflammation deep in joints. As described above, in the case of treatment devices that use ultrasonic waves, there is a limitation in that ultrasonic vibrations are attenuated by bones and may not reach deep inflammation in a cartilage. Since the conventional PEMF therapy uses a pulsed electromagnetic field, it has deep penetration regardless of rigid materials such as bones, but it uses a loop wire-type wire for generating a magnetic force, so it has a defect that the size of a treatment device is large, and high voltage and high current are required. The low-level laser (LLL) therapy has an effect of relieving inflammation, but there is a defect that an extinction phenomenon is generated by reflection and dispersion due to the characteristic of light, so a laser cannot penetrate deep into a joint. The therapy using electrical muscle stimulation (EMS) also has a function of strengthening muscles around a joint without an effect of substantially relieving inflammation.

### [Disclosure]

### [Technical Problem]

An object of an embodiment of the present invention is to provide an arthritis pain treatment device that transmits a magnetic force (or magnetic field) to a deep joint inflamed portion in a bone in a small magnitude by applying a coil-type pulsed electromagnetic generator, enhances the depth of penetration by controlling a low-level laser in a pulse control type, and improves muscle fatigue around a joint and increases muscle strength around a joint by applying an electric muscle stimulation generator and a heat generator, thereby providing a complex function for treating an arthritis pain; and a method of driving the treatment device.

### [Technical Solution]

An arthritis pain treatment device according to an embodiment of the present invention is an arthritis pain treatment device that is worn on a body joint of a user, and includes: a pulsed electromagnetic generator that generates a magnetic field to the body joint to activate cells of the body joint and changes polarity of the magnetic field; a low-level laser generator that generates light of a predetermined wavelength to the body joint in order to reduce inflammation at the body joint and changes brightness of the light; an electric muscle stimulation generator that applies current to muscles of the body joint to induce contraction and relaxation of the muscles of the body joint; a heat generator that generates heat to the muscles around the body joint in order to generate flow of blood (or make flow of blood smooth) around the body joint; and a controller that controls the pulsed electromagnetic generator, the low-level laser generator, the electric muscle stimulation generator, and the heat generator in accordance with instructions from a user.

The pulsed electromagnetic generator includes an electromagnet unit having a pair of coil electromagnets, and the electromagnet unit may transmit magnetic force to a predetermined long distance exceeding a reference value by acting repulsion and attraction of the coil electromagnets.

The coil electromagnet may include: a coil wire wound on a cylindrical frame; a pair of ferromagnetic cores positioned at a center of the coil wire to transmit a magnetic field generated by current, which is applied to the coil wire, in a predetermined direction; one ferromagnetic plate that is disposed on a side of the ferromagnetic core and transmits magnetic force transmitted from the ferromagnetic core to a predetermined long distance; and another ferromagnetic plate that is disposed on another side of the ferromagnetic core and increases magnetic force of the one plate by connecting the pair of ferromagnetic cores.

The one plate may include a first plate and a second plate connected to a side of the pair of ferromagnetic cores, respectively, and the first plate and the second plate may be separated from each other with a predetermined gap.

The another plate may be one plate connecting other sides of the pair of ferromagnetic cores and facing the one plate.

The pulsed electromagnetic generator may modulate a frequency of a pulse signal having predetermined frequency and amplitude to transmit the magnetic force to the predetermined long distance.

The pulsed electromagnetic generator may include at least two electromagnet units and transmit the magnetic force to the predetermined long distance by generating repulsion at the two electromagnet units.

The low-level laser generator may include a conductive guide preventing the light from being exposed to the eyes of a user.

The low-level laser generator may include a first light emitter emitting light of a first wavelength, a second light emitter emitting light of a second wavelength, and a third light emitter emitting light of a third wavelength, in which the wavelengths are different from each other, and operates the first light emitter, the second light emitter, and the third light emitter using a same pulse signal, and may modulate a frequency of the pulse signal when transmitting light to a deep part of the body joint.

Further, a method of driving an arthritis pain treatment device according to an embodiment of the present invention is a method of driving an arthritis pain treatment device that is worn on a body joint of a user, and includes: generating a magnetic field to the body joint to activate cells of the body joint, and changing polarity of the magnetic field by means of a pulsed electromagnetic generator; generating light of a predetermined wavelength to the body joint in order to reduce inflammation at the body joint, and changing brightness of the light by means of a low-level laser generator; applying current to muscles of the body joint to induce contraction and relaxation of the muscles of the body joint by means of an electric muscle stimulation generator; generating heat to the muscles around the body joint in order to generate flow of blood around the body joint by means of a heat generator; and controlling the pulsed electromagnetic generator, the low-level laser generator, the electric muscle stimulation generator, and the heat generator in accordance with instructions from a user by means of a controller.

### [Advantageous Effects]

According to an embodiment of the present invention, it is possible to activate joint cells of a body joint part and reduce inflammation at the joint, enhance the depth of penetration of magnetic force, improve muscle fatigue around a joint, and increase muscle strength around a joint.

### [Description of Drawings]

FIG. 1 is a block diagram exemplifying the detailed structure of an arthritis pain treatment device according to an embodiment of the present invention.
FIG. 2 is a block diagram exemplifying the detailed structure of each of the components of FIG. 1.
FIG. 3 is a plan view of left knee joint application according to an embodiment of the present invention.
FIG. 4 is a side view of left knee joint application.
FIG. 5 is a rear view of left knee joint application (seen in direction A-A).
FIG. 6 is a perspective view of an electromagnet unit of FIG. 2.
FIG. 7 is a cross-sectional view of the electromagnet unit of FIG. 2.
FIG. 8 is an exemplary view of flow of magnetic force by the electromagnet unit of FIG. 2.
FIG. 9 is a front (seen in direction B-B) and cross-sectional view of a medical LED unit of FIG. 2.
FIG. 10 is diagram showing a current signal that is applied to the electromagnet unit of FIG. 2.
FIG. 11 is an H-bridge circuit logic diagram according to an embodiment of the present invention.
FIG. 12 is CAE image showing magnetic force transmission by generation of repulsion by the electromagnet unit of FIG. 2.
FIG. 13 is CAE image showing another magnetic force transmission by generation of attraction of the electromagnet unit of FIG. 2.
FIG. 14 is a diagram showing a pulse signal that is applied to the medical LED unit of FIG. 2.
FIG. 15 is a flowchart showing a process of driving the arthritis pain treatment device of FIG. 1.

### [Best Mode]

Specific structural and functional description about embodiments according to the concept of the present invention disclosed herein is exemplified only to describe the embodiments according to the concept of the present invention, and the embodiments according to the concept of the present invention may be implemented in various ways and are not limited to the embodiments described herein.

The embodiments according to the concept of the present invention may be changed in various ways and various shapes, so specific embodiments are shown in the drawings and will be described in detail in this specification. However, it should be understood that the exemplary embodiments according to the concept of the present invention are not limited to the specific examples, but all of modifications, equivalents, and substitutions are included in the spirit and technical scope of the present invention.

It will be understood that, although the terms first, second, etc. may be used herein to describe various components, these components should not be limited by these terms. These terms are only used to distinguish one element from another element. For instance, a first component could be termed a second element without departing from the right range of the present invention. Similarly, the second element could also be termed the first element.

It is to be understood that when one component is referred to as being "connected to" or "coupled to" another component, it may be connected directly to or coupled directly to another component or be connected to or coupled to another component with the other component therebetween. On the other hand, it should be understood that when one component is referred to as being "connected directly to" or "coupled directly to" another component, no other component exists therebetween. Further, the terms used herein to describe a relationship between components, that is, "between", "directly between", "adjacent to" or "directly adjacent to" should be interpreted in the same manner as those described above.

The terms used herein is only for the purpose of describing particular embodiments and is not intended to limit the present invention. Singular forms include plural forms unless the context clearly indicates otherwise. It should be understood that the term "comprise" or "have" used in this specification, is intended to specify the presence of stated features, steps, operations, components, parts, or a combination thereof, but do not preclude the presence or addition of one or more other features, numerals, steps, operations, components, parts, or a combination thereof.

Unless defined otherwise, all terms including technical and scientific terms used herein have the same meanings as commonly understood by those skilled in the art to which the present invention pertains. It will be further understood that the terms defined in dictionaries that are commonly used should be interpreted as having meanings that are consistent with their meanings in the context of the relevant art and will not be interpreted in an idealized or overly formal sense unless expressly defined herein.

In the drawings, the thicknesses of layers and regions were exaggerated for clarity. When a layer is "on" another layer or a substrate or when a layer is coupled to or bonded to another layer or a substrate, the layer may be formed directly on another layer or a substrate or a third layer may be interposed therebetween. The parts indicated by like reference numerals mean substantially the like components throughout the specification.

The terms such as upper end, lower end, top, bottom, front, rear, or upper portion, lower portion, etc. are used to distinguish relative positions of components. For example, when the upper part is referred to as an upper portion in a drawing and the lower part if referred to as a lower portion in a drawing, the upper portion may be referred to as a lower portion and the lower portion may be referred to as an upper portion in actual cases without departing from the scope of the present invention. Further, the components in the drawings are not shown necessarily in accordance with scales, and for example, the sizes of some components may be exaggerated in comparison to other components to help understand the present invention.

Hereinafter, the embodiments of the present invention are described in detail with reference to the accompanying drawings.

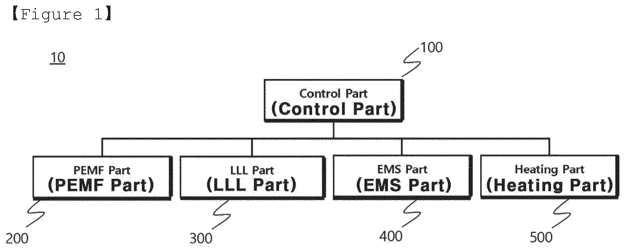

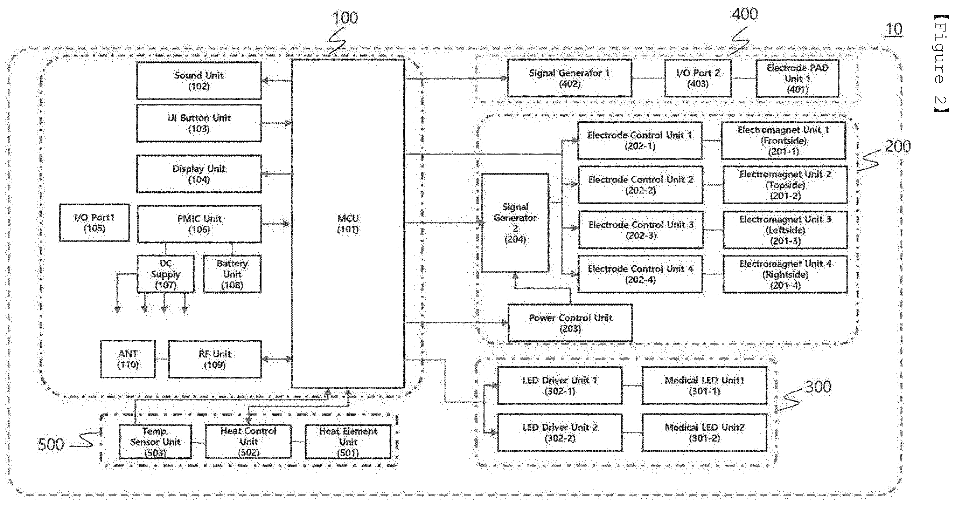

FIG. 1 is a block diagram exemplifying the detailed structure of an arthritis pain treatment device according to an embodiment of the present invention, and FIG. 2 is a block diagram exemplifying the detailed structure of each of the components of FIG. 1.

As shown in FIG. 1, an arthritis pain treatment device 10 according to an embodiment of the present invention includes some or all of a controller 100, a pulsed electromagnetic generator 200, a low-level laser generator 300, an electric muscle stimulation generator 400, and a heat generator 500.

The term "including some or all" means that the arthritis pain treatment device (hereafter, treatment device) 10 is configured without some components such as the heat generator 500 or some components such as the pulsed electromagnetic generator 200 are included in other components such as the controller 100, and it is exemplified in the following description that all of them are included to help sufficiently understand the present invention.

The controller 100 is in charge of generally controlling the pulsed electromagnetic generator 200, the low-level laser generator 300, the electric muscle stimulation generator 400, and the heat generator 500. The pulsed electromagnetic generator 200, the low-level laser generator 300, the electric muscle stimulation generator 400, and the heat generator 500 correspond to treatment modules, respectively, so, for example, when an instruction is given by a user, the controller 100 controls each component on the basis of the instruction of user. The controller 100, as shown in FIG. 2, includes some or all of an MCU 101, a sound generation unit 102, a user button unit 103, a display unit 104, an in/out port (or first in/out port) 105, a power management integrated circuit unit (or power management unit) 106, a converter unit 107, a battery unit 108, a wireless communication unit 109, and an antenna unit 110, and, in this case, the term "including some or all" is not much different from the meaning described above. A unit may be referred to as a device or a part, and the in/out port 105 may be referred to as an I/O port. Further, the term "part" may be used as a meaning that includes a plurality of circuits.

In more detail, the controller 100 is composed of an MCU (or CPU) 101 that manages and controls the operation scenarios of the entire HW system and each of the units of the treatment device 10, a sound generation unit 102 that informs a user of the operation state of the treatment device 10 in a sound type such as a speaker or a buzzer, a user button unit 103 that enables a user to select operation options, etc. using a button, or the like, a display unit 104 that displays the operation state of the treatment device, selective options for a user, etc. on a screen, an input/output port 105 that is connected with the outside for charging and controlling, a power management integrated circuit (PMIC) unit 106 that receives power input to the input/output port 105 and supplies power to a battery charging integrated circuit (IC) or distributes appropriate power to all of ICs of the HW system, a converter unit 107 that supplies power at a predetermined voltage to various parts of the HW system by stepping down or stepping up power applied from the PMIC, a battery unit 108 that stores electrical energy to supply power to the internal HW circuit without a specific external power, a wireless communication unit 109 that can remotely control the treatment device 10 through an external wireless device such as a remote controller and a smartphone, and an antenna unit 110 for transmitting signals of the wireless communication unit 109. When a user operates the MCU 101 by applying power to the entire system using the user button 103, the MCU 101 controls and starts a peripheral HW IC. A user may set desired treatment modes and intensity and operate the device through the user button, and the operation state of the device and the selections by a user are displayed to the user through the display unit 104. An abnormal situation in operation is represented through a voice or a sound effect by the sound unit 102. The wireless communication unit 109 may perform infrared communication, but may perform near field communication such as Bluetooth or ZigBee.

The pulsed electromagnetic generator 200, as shown in FIG. 2, includes some or all of an electromagnet unit 201, an electrode control unit 202, a power control unit 203, and a pulse signal generation unit 204, and, in this case, the term "including some or all" is not much different from the meaning described above. The electromagnet unit 201 generates a magnetic field when current is applied, and the electrode control unit 202 induces an AC-type waveform by changing the magnetic polarity of the electromagnet unit 201 by changing the direction of current that is applied to the electromagnet unit 201. Further, the power control unit 203 controls the magnitude of electromagnetic force by controlling the amounts of voltage and current that are applied to the electromagnet unit 201, and the pulse signal generation unit 204, that is, the pulse signal generator generates a pulse signal so that the characteristics of predetermined pulse and frequency (Hertz) are shown in a magnetic force variation form of the electromagnet unit 201. The electromagnet unit 201 according to an embodiment of the present invention may include a plurality of electromagnet units and may include first to fourth electromagnet units 201-1 ~ 201-4. The electromagnet units may be provided to be positioned at the upper, lower, left, and right sides with respect to a knee of a user. The pulsed electromagnetic generator 200 operates to activate the cells at a joint.

The low-level laser generator 300 includes some or all of a medical LED unit (or a light emitter) 301 and an LED control unit 302. The medical LED unit 301 generates a light (optical) wavelength for treatment and the LED control unit 302 changes brightness (or intensity) by changing the amount of current applied to the medical LED unit 301 or maintains LED brightness constant by maintaining the amount of current constant. For the low-level laser generator 300 according to an embodiment of the present invention, an IR LED having three kinds of wavelengths of 650nm, 808nm, 980nm may be applied, and IR LEDs of various wavelengths may be applied depending on the treatment area. The LED wavelength of the low-level laser generator 300 is operated to reduce inflammation of a joint part.

The electric muscle stimulation generator 400 includes some or all of a current polarity pad 401, an AC signal generation unit 402, and an input/output port 403. The current polarity pad 401 is attached to a skin and induces current to be able to be transmitted to a muscle. The AC signal generation unit 402 generates an AC signal so that current, which is applied to the current polarity pad 401 for contraction and relaxation of muscles, shows characteristics of predetermined pulse and frequency (Hertz). The input/output port (or a second input/output port) 403 is a port that can connect the current polarity pad 401 at a position outside the product, if necessary. The electric muscle stimulation generator 400 is operated to increase the strength of muscles around a joint.

The heat generator 500 includes some or all of a heating unit 501, a heat control unit 502, and a sensing unit 503. The heating unit 501 induces a heating effect by generating heat on a skin contact surface during a treatment process. Further, the heat control unit 502 serves to apply predetermined current to the heating unit 501 and control the current. The sensing unit 503 senses temperature at the heating unit 501. The sensing unit 503 may include a temperature sensor, etc. The heat control unit 502 can be operated under the control of the MCU 101. The heat generator 500 is operated to help blood circulation by generating minute heat in the muscles around a joint.

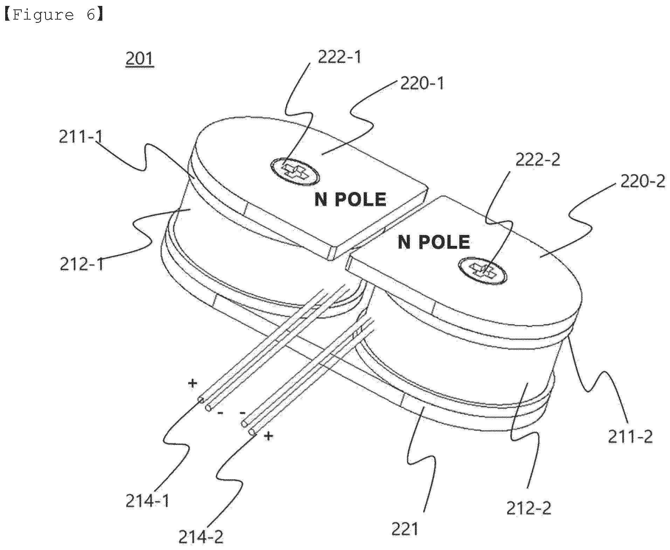

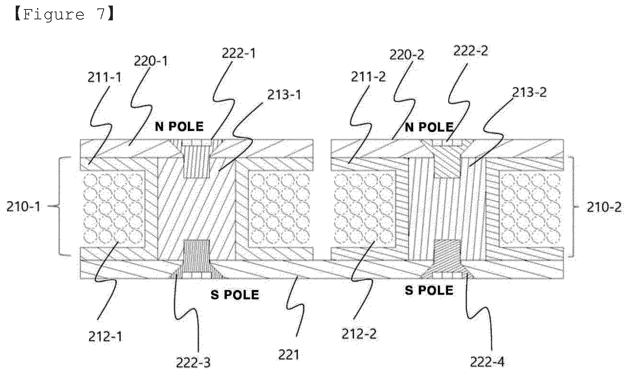

In short, the arthritis pain treatment device 10 according to an embodiment of the present invention includes a pulsed electromagnetic generator 200 that activates cells at a joint, a low-level laser generator that reduces arthritis inflammation, an electric muscle stimulation generator 400 that increases the strength of the muscles around a joint, and a heat generator 500 that helps blood circulation by generating minute heat in the muscles around a joint. In this configuration, the electromagnet unit 201 of the pulsed electromagnetic generator 200, as shown in FIG. 6 and FIG. 7, has coil electromagnets 210 in a pair, so it can transmit magnetic force to a long distance by acting repulsion and attraction of the coil electromagnets 210. In this case, the long distance may be determined as a distance exceeding a reference value. In this configuration, the electromagnet unit 201 includes: a coil electromagnet 210 composed of a cylindrical frame 211 on which a coil wire 212 can be wound in a predetermined shape, a coil wire 212 made of a conductive material such as copper to generate a magnetic field when current is applied, a ferromagnetic core 213 positioned at the center of the coil to transmit a magnetic field generated at the coil wire in a predetermined direction, a coated insulating cable 214 connected to both ends of the coil wire 212 and receiving positive and negative currents; a ferromagnetic inner plate 220 supposed to face a joint of a person to transmit magnetic force, which is transmitted from the ferromagnetic core 213, to a long distance; a ferromagnetic outer plate (or a second plate) 221 applied to the opposite side to the inner plate 220 and increasing magnetic force of the inner plate (or a first plate) 220 by connecting the electromagnetic cores 213 applied in a pair; and a fixing member 222, such as a screw, for fixing the inner plate 220 and the outer plate 221 to the core 213 of the electromagnet unit. In this configuration, the term `inner' means a part of the user's body joint that is in contact with (or approaches) the skin.

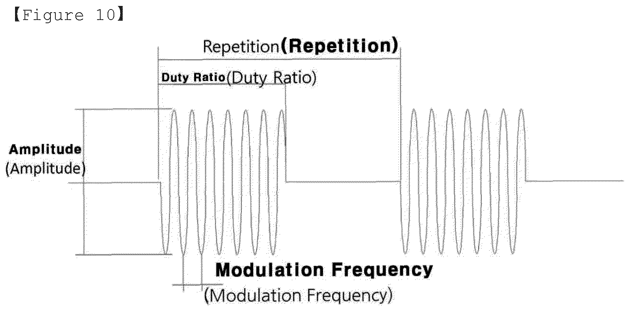

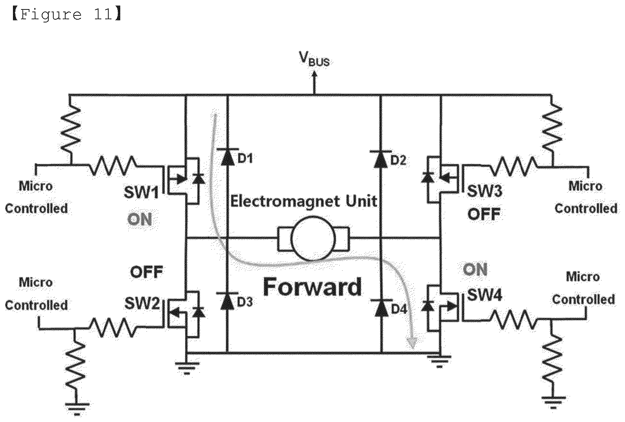

The inner plate 220 connecting the cores 213 of the coil electromagnet 210 in the electromagnet unit 201 is configured with a short distance of 1~ 2mm, and for example, is composed of two plates separated from each other with a predetermined gap, and the outer plate 221 connecting the cores 213 of the coil electromagnet 210 in the electromagnet unit 201 is connected as a single body at a shortest distance without a gap, shows a magnetic force type showing the characteristic of a pulse signal to transmit magnetic force to a long distance, and to this end, may include a unit 204 that generates a pulse signal and an electrode control unit 202 that changes a magnetic polarity in an H-bridge type. This is shown well in FIG. 10 and FIG. 11. Referring to FIG. 11, an H-bridge is configured such that first to fourth switching elements form an H-shape around an electromagnet unit. The switching elements are each composed of an NPN (or N-channel) element and a PNP (or P-channel) element of a transistor or an FET. Further, the MCU 101 of FIG. 2 can alternately operate the switching elements so that opposite polarities are generated at the electromagnet unit by applying a LOW signal and a HIGH signal to the first switching element and the fourth switching element or applying a LOW signal and a HIGH signal to the third switching element and the second switching element. That is, it may be considered as changing magnetic polarities by complementarily operating switching elements diagonally facing each other.

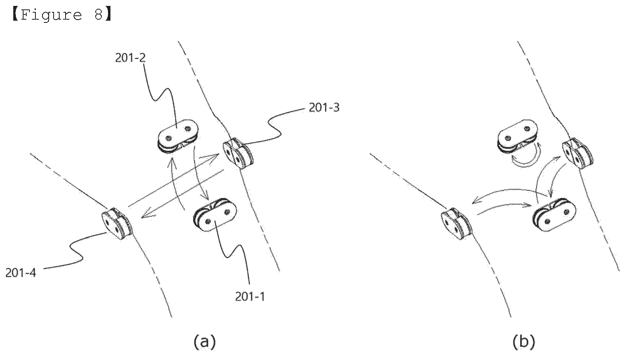

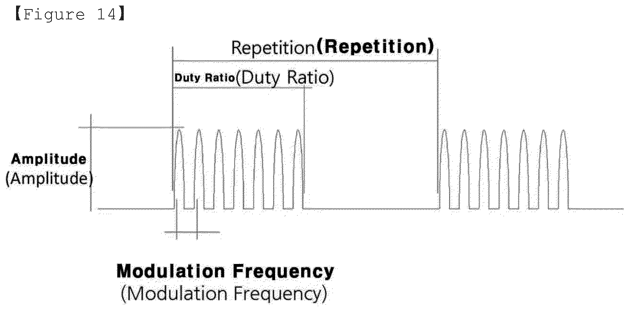

Further, the pulse that is applied to the electromagnet unit 201 may be configured as a carrier signal type to transmit magnetic force to a long distance, two or more electromagnet units 201 are applied to the treatment device such that repulsion is generated at the inner plate 220 of one electromagnet unit and repulsion of the opposite polarity is generated at the opposite electromagnet unit, whereby it is possible to transmit magnetic force to a longer distance. This is shown well in FIG. 8. In this case, the "pulse is configured as a carrier signal type" means that, referring to FIG. 10 and FIG. 14, a new pulse is created and used by splitting a pulse signal (e.g., an operation frequency) generated with predetermined frequency and amplitude, more accurately, splitting a pulse generation period. It may be considered as modulating and then using a preset pulse signal to transmit magnetic force to a long distance and make light permeate into a deep part. For example, it is possible to convert a pre-created pulse signal of 20 ~ 50Hz into a signal of about 2 MHz and then use the signal.

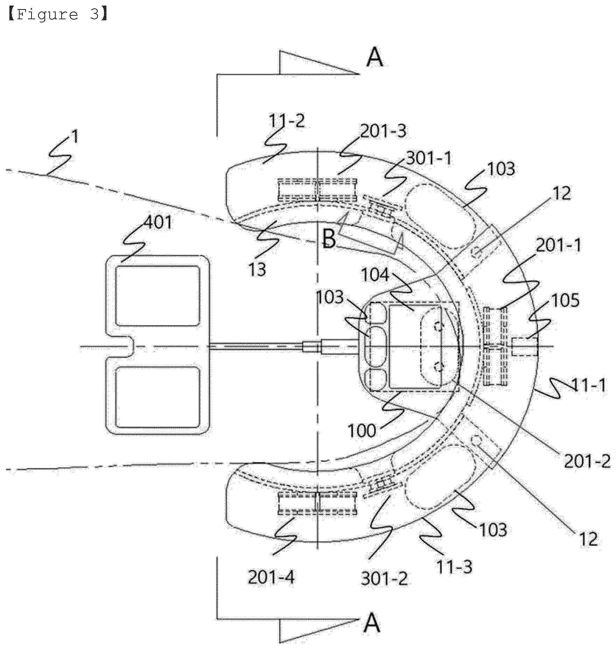

Further, in the arthritis pain treatment device according to an embodiment of the present invention, conductive guides 303 may be spaced up and down or left and right to prevent light of the low-level laser generator 300 from being emitted directly to the eyes of a user, the medical LED uses wavelengths of 650nm, 808nm, and 980nm and outputs light in a pulse type to increase the permeation depth, and the output pulse of the medical LED is configured as a carrier signal type to increase the deep permeation depth of IR light. Further, three kinds of medical LEDs simultaneously output light in a same pulse type to make output IR light permeate up to a deep part. Meanwhile, the current polarity pad 410 may be connected with the controller 100 at the outside through a cable 404 and the input/output port 403 in order to reduce the size of the treatment device and efficiently enhance muscles, and a case may be divided into a center case 11-1 and side cases 11-1 and 11-2 and a rotatable hinge 12 may be applied to the connected portion in order to apply to various joint parts. This is shown well in FIG. 3.

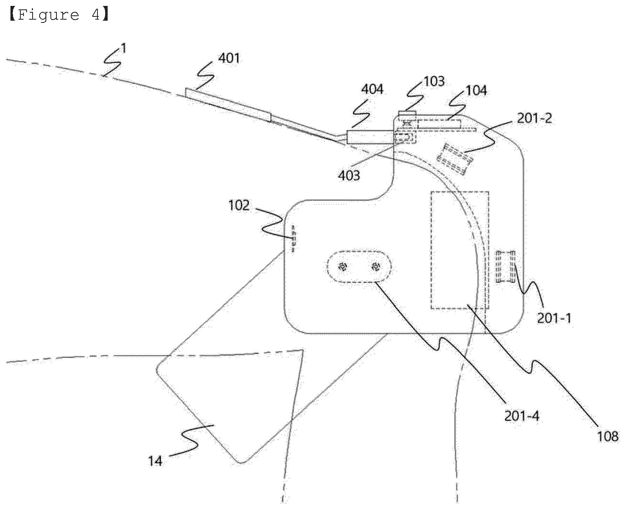

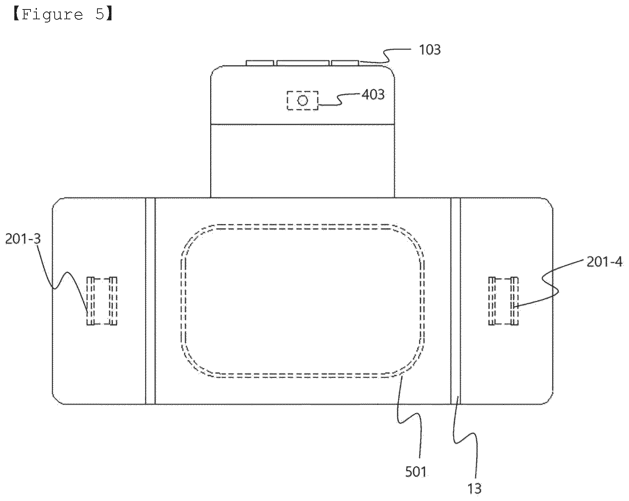

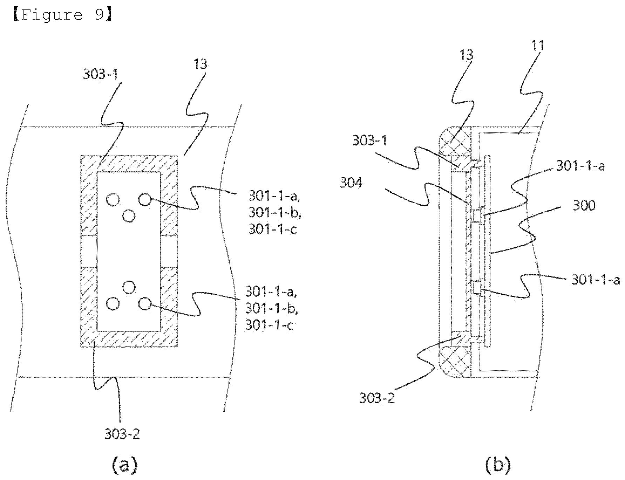

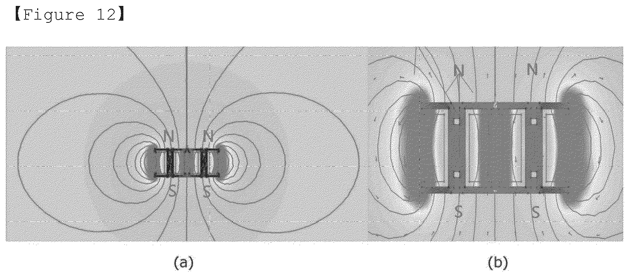

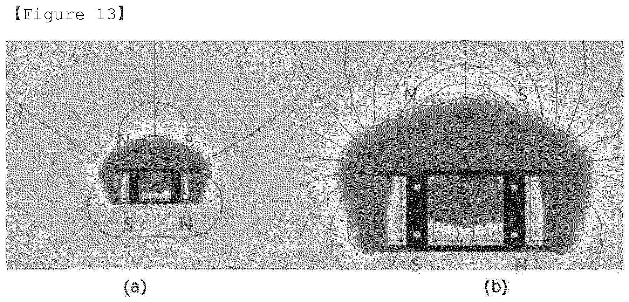

FIG. 3 is a plan view of left knee joint application according to an embodiment of the present invention, FIG. 4 is a side view of left knee joint application, FIG. 5 is a rear view of left knee joint application (seen in direction A-A), FIG. 6 is a perspective view of an electromagnet unit of FIG. 2, FIG. 7 is a cross-sectional view of the electromagnet unit of FIG. 2, FIG. 8 is an exemplary view of flow of magnetic force by the electromagnet unit of FIG. 2, FIG. 9 is a front (seen in direction B-B) and cross-sectional view of a medical LED unit of FIG. 2, FIG. 10 is diagram showing a current signal that is applied to the electromagnetic unit of FIG. 2, FIG. 11 is an H-bridge circuit logic diagram according to an embodiment of the present invention, FIG. 12 is CAE image showing magnetic force transmission by generation of repulsion by the electromagnet unit of FIG. 2, FIG. 13 is CAE image showing another magnetic force transmission by generation of attraction by the electromagnet unit of FIG. 2, and FIG. 14 is a diagram showing a pulse signal that is applied to the medical LED unit of FIG. 2.

Referring to FIG. 3, the reference numeral '1' indicates a knee of a person, '11-1' indicates the center case, '11-2' indicates the left side case, '11-3' indicates the right side case, '12' indicates a rotary hinge unit, and '13' indicates a cushion pad unit. Further, referring to FIG. 4, the reference numeral '404' indicates a cable for connecting an external current polarity pad 401 and '14' indicates a fixing belt unit. Further, in FIG. 6 and FIG. 7, the reference numeral '210' indicates a coil magnet generating magnetic force through a coil wire, '211' indicates a cylindrical frame on which the coil wire can be wound in a predetermined shape, '212' indicates a copper coil wire for generating a magnetic field when current is applied, '213' indicates a ferromagnetic core positioned at center of the coil to transmit a magnetic field, which is generated at the copper coil wire, in a predetermined direction, '214' indicates a coated insulating cable connected with both ends of the coil wire and receiving positive (+) current of DC power, '220' indicates a ferromagnetic inner plate supposed to face a joint of a person to transmit magnetic force, which is transmitted from the ferromagnetic core 213, to a long distance, '221' indicates a ferromagnetic outer plate applied to the opposite surface to the ferromagnetic inner plate 220 and increasing the magnetic force of the inner plate by connecting a pair of cores 213 of the coil electromagnet 210 disposed in the electromagnet unit 201, and '222' indicates a fixing member, such as a screw, for fixing the inner plate and the outer plate to the cores 213 of the electromagnet unit. Referring to FIG. 9, the reference numeral '303' indicates a conductive guide preventing light generated from the medical LED 301 from leaking during a treatment process and preventing the eyes of a user from being exposed to the light due to carelessness, '301-1-a' indicates a medical LED having a wavelength value 'a', '301-1-b' indicates a medial LED having a wavelength value 'b', '301-1-c' indicates a medial LED having a wavelength value 'c', and '304' indicates a protective cover protecting an IR LED from external shock and transmitting light.

For the convenience of description, referring to FIG. 3 to FIG. 14 together with FIG. 1 and FIG. 2, the pulsed electromagnetic generator 200 of FIG. 1, as shown in FIG. 2, FIG. 6 and FIG. 7, is composed of an electromagnet unit 201 generating a magnetic field, an electrode control unit 202 inducing an AC-type waveform by changing the magnetic polarity of the electromagnet unit, a power control unit 203 controlling the amount of voltage and current that are applied to the electromagnet unit 201, and a unit 204 generating a pulse signal including the amplitude, cycle, duty ratio, and carrier frequency of a magnetic filed, as shown in FIG. 10, for effective treatment. That is, a signal generation unit 204 that generates a preset pulse signal for treatment may be included.

When a user selects intensity and a treatment type to drive the treatment device 10 through the user button 103, the MCU 101 correspondingly changes the intensity of magnetic force by controlling a current value by changing a voltage value of the power control unit 203 and controls the signal generation unit 204, thereby generating a pulse signal fitted to the treatment purpose. Of course, this operation may be performed by setting corresponding values in advance. A lookup table (LUT) may be used in this process. Further, a pulse signal is changed into an AC signal by controlling the electrode control unit 202. It is preferable that the electrode control unit 202 is configured as an H-bridge circuit logic, as shown in FIG. 11, the direction of current that is applied to the electromagnet unit 201 is changed by control by the MCU 101, and accordingly, the polarity of the inner plate 220 of the electromagnet unit 201 is also changed. The H-bridge circuit can be operated in various ways in accordance with an embodiment of the present invention, so it would not be specifically limited to any one operation type (e.g., a complementary operation).

The electromagnet unit 201 includes a pair of coil electromagnets 210 therein. For example, two, that is, a pair of coil electromagnets 210-1 and 210-2 is applied to the electromagnet unit 20, as in FIG. 6 and FIG. 7. The coil electromagnets 210 are connected to the electrode control unit 202 by winding a coil on a nonmagnetic cylindrical frame 211 several times in the type of wire 212 and applying a cable 214 coated with an insulating material such as poly-based PVC to the end of the coil. It is preferable to apply a nonmagnetic material such as plastic as the material of the cylindrical frame 211.

Further, the ferromagnetic cores 213 are applied to the center of the wound coli and perform a function of transmitting a magnetic field, which is generated at the coil wire (or wound coil) 212, in a predetermined direction. That is, the magnetic polarities at both ends of the ferromagnetic cores 213 are changed into N-S pole or S-N pole, depending on the direction of current applied to the insulating cables 214 connected to both ends of the coil wire 212. The inner plate (or first plate) 220 is connected toward a human body to both ends of the ferromagnetic cores 213 of the pair of coil electromagnet 210 adjacent to each other with a predetermined gap using fixing members 222-1 and 222-2, and the outer plate (or second plate) 221 is connected and fixed to the opposite direction of the inner plate using fixing members 222-3 and 222-4.

Since the purpose is to transmit magnetic force to a long distance by transmitting the magnetic force of the cores 213 to the inner and outer plates 221 and 222, a ferromagnetic material having high specific permeability is applied to each of the plates and the fixing members. In this case, specific permeability means the ratio of magnetization, and when the ratio to magnetic force transmission permeability in vacuum, that is, this value is several hundreds to several thousands, it is called a ferromagnetic material.

Substantially, as shown in FIG. 6, when current is applied with + and - polarities to the insulating cable 214-1 of the coil electromagnet 210-1 positioned at the left of the electromagnet unit 201 and current of + and - polarities is applied to the insulating cable 214-2 of the right coil electromagnet 201-2, an N pole is inducted at the inner plate 220-1 of the left coil electromagnet 210-1 and an N pole is induced at the inner plate 220-2 of the right coil electromagnet 210-2 according to Ampere's right-hand rule.

In this case, the directions of the magnetic force applied to the two inner plates are the same, as in the CAE simulation analysis result shown in FIG. 12, so repulsion is generated therebetween, and accordingly, it is possible to transmit magnetic force to a long distance.

If current is applied with + and - polarities to the insulating cable 214-2 of the right coil electromagnet 210-2, an S pole is induced at the inner plate 220-2 of the right coil electromagnet and attraction is generated between the inner plates, so a force pulling each other is generated. When attraction is generated in this way, it can be seen that magnetic force generated between the inner plates is also transmitted to a long distance, as in the CAE simulation analysis result shown in FIG. 13, because each of the cores 213-1 and 213-2 is connected through the outer plate 221.

The number and position of the electromagnet unit 201 that is applied may be different and may be changed in various ways, depending on the anatomical features of joints to be treated. When an embodiment of the present invention is applied to knee joint pain treatment, it is preferable to position a front side 201-1, a plane 201-2, a left side 201-3, and a right side 201-4 to be able to face the articular cartilage at the lower end of the femur and the meniscus cartilage of the shinbone that are easily inflamed by wear of the cartilage, as in FIG. 2 to FIG. 5, and FIG. 8.

When the electromagnet units 201 are disposed, as described above, and the magnetic force and the polarity of each of the electromagnet units 201 are changed, magnetic force penetrates an articular inflamed part in various direction and various intensities, as in FIG. 8.

In particularly, it can be seen that when attraction is generated between electromagnet units 202 facing each other and receiving current to generate repulsion, magnetic force can reach a longer distance.

Meanwhile, the low-level laser generator 300 is, as in FIG. 2, FIG. 3, and FIG. 9, composed of a medical LED unit 301 that generates light of a specific wavelength and an LET control unit 302 that controls brightness by changing the amount of current of the LED unit.

It is preferable to apply an IR LED having two or more waveforms as the medical LED unit 301. An IR LED having three kinds of wavelengths of 650nm, 808nm, and 980nm was applied to the present invention, but IR LEDs having various wavelengths may be applied, depending on the parts to be treated. The number and position of the IR LED unit 301 may be different and may be changed in various ways, depending on the anatomical features of joints to be treated.

When an embodiment of the present invention is applied to knee joint pain treatment, it is preferable to dispose an IR LED at the left and right of a kneepan because kneepans extinguish light due to the translucent material characteristic, and it is preferable to distribute an IR LED and dispose the IR LED 301 having three kinds of wavelengths at upper and lower ends, respectively, as in FIG. 9, in consideration of the size and height of a femur joint.

Further, it is preferable to apply an LED that generates light in a low-level laser type. The LED control unit 302, as in FIG. 14, performs a function of generating a pulse signal, to which a voltage amplitude, a cycle, a duty ratio, and a carrier frequency have been applied, for effective treatment. The protective cover 304 that transmits light is applied to the front side to protect the medical LED unit 301 and a conductive guide 303 for preventing exposure of light is disposed around the protective cover. It is preferable that the conductive guide 303 is a conductive silicone rubber type that is in contact with the medical LED unit 301. The conductive guide is configured at two positions of upper and lower portion or left and right sides, when the conductive guide comes in contact with a skin, the capacitance value of the medical LED unit 301 is changed, and accordingly, the controller can determine whether there is a problem in the wearing state of the device on a user. If both the conductive guides 303 at two positions are not in contact with a skin, it is the case when a user does not correctly wear the device, and in this case, the controller 100 represents the error through the display unit 104 and the sound unit 102 without starting the corresponding function to prevent a low-level laser from being emitted to the eyes of a user.

The electric muscle stimulation generator 400, as shown in FIG. 2, FIG. 3, and FIG. 4, is composed of a current polarity pad 401 that induces contraction and relaxation of muscles by being attached to a skin and applying current to muscles, a pulse signal generation unit 402 that supplies AC current having a pulse-type waveform to the pad, and an input/output port 403 for connection to the controller 100 in a wire type at the outside of the treatment device. It is also required to strengthen surrounding muscles together with reduction of inflammation and activation of cells of a cartilage in order to reduce arthritis pain, and for this purpose, it is preferable to attach the current pole pad 401 to the region right over a knee at which the vastus medialis and the vastus lateralis are positioned. To this end, the input/output port 403 is positioned toward corresponding muscle parts and the current pole pad 401 may be connected through a cable 404.

The heat generator 500 has a purpose of making flow of blood smooth by providing heat to the muscles around a knee during a treatment process as in an embodiment of the present invention, and is composed of a heating unit 501 that generates heat, a current control unit 502 that controls the amount of current applied to the heating unit 501, and a temperature sensing unit 503 that can maintain temperature that a user wants by measuring the temperature of the heating unit 501.

As in FIG. 5, it is preferable that the heating unit 501 is applied to a wide region, which comes in contact with the muscles around a joint, in the treatment device. Further, in order to prevent a low-temperature burn due to the heating unit 501, it is preferable that a cushion pad 13 is fixed therein to maintain gap in some extent from a skin and the heating unit is driven for a relatively short time within 10 to 20 minutes at a temperature of 40 to 50 degrees.

The treatment device 10, as shown in FIG. 3, is divided into a center case 11-1 fixing units such as the front and plan electromagnet units 201 and the controller 100, and side cases 11-2 and 11-3 fixing the left electromagnet unit 201-3 and the medical LED unit 201, in which the center case and the side cases constitute the external case 11 so that the units are firmly fixed. The side cases 11-2 and 11-3 and the center case 11-1 have a hinge structure 12 to be able to be applied to various sizes of joint parts. It is preferable to apply a fixing belt unit 14 in order to prevent separation of the device during a treatment process when the treatment device 10 is mounted on joints such as a knee, a shoulder, and an elbow.

The treatment processes described above may be simultaneously performed and a single therapy or two to three therapies may be simultaneously performed, depending on treatment purposes of users. It is preferable that one-time treatment operation is within 10 to 20 minutes and the device is used one to two times a day.

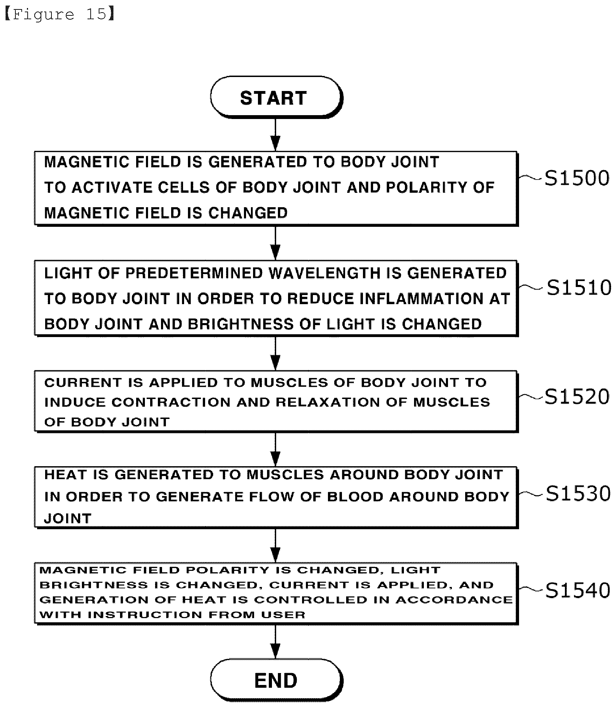

FIG. 15 is a flowchart showing a process of driving the arthritis pain treatment device of FIG. 1.

For the convenience of description, referring to FIG. 15 together with FIG. 1, the arthritis pain treatment device 10 according to an embodiment of the present invention generates a magnetic field to a body joint in order to activate the cells of the body joint, and changes the polarity of the magnetic field (S1500). Of course, this operation may be performed at the pulsed electromagnetic generator 200 of FIG.

1. The pulsed electromagnetic generator 200 preferably includes electromagnetic units, for example, disposed at the upper and lower portions and left and right sides of a knee, and generates repulsion or attraction between each electromagnetic unit and an adjacent electromagnetic unit. Further, even each electromagnetic unit itself includes two coil electromagnets, so attraction or repulsion is also generated between the coil electromagnets. Details were sufficiently described above, so the details will be replaced by those contents. Of course, such control may be performed by the controller 100 in accordance with a preset method.

Further, the arthritis pain treatment device 10 generates light of a predetermined wavelength to a body joint in order to reduce inflammation at the body joint, and changes the brightness (or intensity) of the light (S1510). This operation may be performed at the low-level laser generator 300 of FIG. 1. In accordance with an embodiment of the present invention, the low-level laser generator 300 may include, for example, three light emitters, that is LEDs, and operate the light emitters to generate light with different wavelengths, but may operate a plurality of light emitters using a same pulse signal so that output light permeates into a deep part.

Further, the arthritis pain treatment device 10 applies current to muscles of a body joint to induce contraction and relaxation of the muscles of the body joint (S1520). This is performed by the electric muscle stimulation generator 400, and may be implemented by putting electrodes on a joint part and then applying fine current to corresponding electrodes or electrode pads.

Furthermore, the arthritis pain treatment device 10 generates heat to the muscles around a body joint in order to generate flow of blood (or make flow of blood smooth) around the body joint (S1530).

In addition, the arthritis pain treatment device 10 may receive instructions from a user through the user button of the arthritis pain treatment device 10, and controls the pulsed electromagnetic generator 200, the low-level laser generator 300, the electric muscle stimulation generator 400, and the heat generator 500 in accordance with corresponding instructions (S1530). The components may be operated individually, sequentially, or simultaneously, depending on control by the controller 100, and this is referred to as "selectively operated" in an embodiment of the present invention. Of course, they may be operated in a first type in accordance with a first instruction and may be operated in a second type in accordance with a second instruction, and when a plurality of operations sequentially performs a series of operations in accordance with one instruction, it may be referred to as a mode type.

As described above, various operation types of the components may be implemented through the controller 100, so the operation type of the component is not limited to any one specific type in an embodiment of the present invention.

Meanwhile, it is shown and described that the controller 100 and various components for treatment of arthritis are included in an embodiment of the present invention, but this may be freely changed. In other words, as another embodiment of the present invention, it is also possible to freely implement a configuration including the controller 100 and an arthritis treatment part. Of course, the arthritis treatment part in this case may include at least one component of the pulsed electromagnetic generator 200, the low-level laser generator 300, the electric muscle stimulation generator 400, and the heat generator 500 of FIG. 1. Of course, when all of them are included, they may be integrated. Accordingly, various types of configurations are possible in an embodiment of the present invention, so the present invention would not be limited specifically to the configuration of FIG. 1.

Although preferred embodiments of the present invention were illustrated and described above, the present invention is not limited to the specific embodiments above, and may be modified in various ways by those skilled in the art without departing from the scope of the present invention described in claims, and the modified examples should not be construed independently from the spirit of the scope of the present invention.

Meanwhile, even through all components constituting the embodiments of the present invention are combined in one unit or operated in combination in the above description, the present invention is not limited thereto. That is, the all components may also be selectively combined and operated within the scope of the present invention. Further, although all the components may be implemented as individual hardware, some or all of the components may be selectively combined and implemented as a computer program having program modules that perform some or all of functions combined in one or several items of hardware. Codes and code segments of the computer programs may be easily inferred by those skilled in the art. The computer programs may be stored in a non-transitory computer readable media and read out and executed by a computer, thereby achieving embodiments of the present invention.

The non-transitory computer readable media is not a media that stores data for a short time such as a register, a cache, and a memory, but a media that can semipermanently store data and can be read out by a device. In detail, the programs may be stored and provided in a non-transitory computer readable media such as a CD, a DVD, a hard disk, a blueray disc, a USB, a memory card, and a ROM.

Although preferred embodiments of the present invention were illustrated and described above, the present invention is not limited to the specific embodiments above and may be modified in various ways by those skilled in the art without departing from the gist of the present invention described in claims, and the modified examples should not be construed independently from the technical idea or the perspective of the present invention.

## Claims

1. An arthritis pain treatment device that is worn on a body joint of a user, the arthritis pain treatment device comprising:
a pulsed electromagnetic generator (200) that generates a magnetic field to the body joint to activate cells of the body joint and changes polarity of the magnetic field;
a low-level laser generator (300) that generates light of a predetermined wavelength to the body joint in order to reduce inflammation at the body joint and changes brightness of the light;
an electric muscle stimulation generator (400) that applies current to muscles of the body joint to induce contraction and relaxation of the muscles of the body joint;
a heat generator (500) that generates heat to the muscles around the body joint in order to generate flow of blood around the body joint; and
a controller (100) that controls the pulsed electromagnetic generator (200), the low-level laser generator (300), the electric muscle stimulation generator (400), and the heat generator (500) in accordance with instructions from a user.

2. The arthritis pain treatment device of claim 1, wherein the pulsed electromagnetic generator (200) includes an electromagnet unit (201) having a pair of coil electromagnets (210), and
the electromagnet unit (201) transmits magnetic force to a predetermined long distance exceeding a reference value by acting repulsion and attraction of the coil electromagnets (210) .

3. The arthritis pain treatment device of claim 2, wherein the coil electromagnet (210) includes:
a coil wire (212) wound on a cylindrical frame;
a pair of ferromagnetic cores (213) positioned at a center of the coil wire (212) to transmit a magnetic field generated by current, which is applied to the coil wire (212), in a predetermined direction;
one ferromagnetic plate (220) that is disposed on a side of the ferromagnetic core (213) and transmits magnetic force transmitted from the ferromagnetic core (213) to a predetermined long distance; and
another ferromagnetic plate (221) that is disposed on another side of the ferromagnetic core (213) and increases magnetic force of the one plate (220) by connecting the pair of ferromagnetic cores (213).

4. The arthritis pain treatment device of claim 3, wherein the one plate (220) includes a first plate and a second plate connected to a side of the pair of ferromagnetic cores (213), respectively, and the first plate and the second plate are separated from each other with a predetermined gap.

5. The arthritis pain treatment device of claim 4, wherein the another plate (221) is one plate connecting other sides of the pair of ferromagnetic cores (213) and facing the one plate (220) .

6. The arthritis pain treatment device of claim 2, wherein the pulsed electromagnetic generator (200) transmits the magnetic force to the predetermined long distance by modulating a frequency of a pulse signal having predetermined frequency and amplitude.

7. The arthritis pain treatment device of claim 2, wherein the pulsed electromagnetic generator (200) includes at least two electromagnet units (201) and transmits the magnetic force to the predetermined long distance by generating repulsion at the two electromagnet units (201).

8. The arthritis pain treatment device of claim 1, wherein the low-level laser generator (300) includes a conductive guide (303) preventing the light from being exposed to the eyes of a user.

9. The arthritis pain treatment device of claim 8, wherein the low-level laser generator (300) includes a first light emitter emitting light of a first wavelength, a second light emitter emitting light of a second wavelength, and a third light emitter emitting light of a third wavelength, in which the wavelengths are different from each other, and operates the first light emitter, the second light emitter, and the third light emitter using a same pulse signal, and modulates a frequency of the pulse signal when transmitting light to a deep part of the body joint.

10. A method of driving an arthritis pain treatment device that is worn on a body joint of a user, the method comprising:
generating a magnetic field to the body joint to activate cells of the body joint, and changing polarity of the magnetic field by means of a pulsed electromagnetic generator (200);
generating light of a predetermined wavelength to the body joint in order to reduce inflammation at the body joint, and changing brightness of the light by means of a low-level laser generator (300);
applying current to muscles of the body joint to induce contraction and relaxation of the muscles of the body joint by means of an electric muscle stimulation generator (400);
generating heat to the muscles around the body joint in order to generate flow of blood around the body joint by means of a heat generator (500); and
controlling the pulsed electromagnetic generator (200), the low-level laser generator (300), the electric muscle stimulation generator (400), and the heat generator (500) in accordance with instructions from a user by means of a controller (100).
